# EUROPEAN PATENT APPLICATION

(11) **EP 0 551 169 A1**
(43) Date of publication of application: **14.07.1993**
(21) Application number: 93300007.7
(22) Date of filing: 04.01.1993
(51) Int. Cl.: A61K 9/127

(54) **Liposome composition and production thereof**

(30) Priority: 10.01.1992 JP 3363/92
(71) Applicant: TAKEDA CHEMICAL INDUSTRIES, LTD., Chuo-ku, Osaka 541 (JP)
(72) Inventor: Yamamoto, Masaki, Takatsuki, Osaka 569 (JP); Morikawa, Kumiko, Suita, Osaka 565 (JP); Suzuki, Yasuyuki, Suita, Osaka 565 (JP)
(74) Representative: Dowden, Marina

(57) **Abstract**

A liposome composition in which an aqueous solution containing a water-soluble drug in saturated concentration or more at ordinary temperature is encapsulated in liposomes is disclosed. The liposome composition is produced by forming a liposome membrane under warming by using as a solution to be encapsulated an aqueous solution containing a water-soluble drug in saturated concentration or more at ordinary temperature.

## Description

### FIELD OF THE INVENTION

The present invention relates to a liposome composition, in which a water-soluble drug is encapsulated in high concentration within liposomes, and a process for its production.

### BACKGROUND OF THE INVENTION

Utilization of liposomes has extensively been studied in the field of medicaments, agricultural chemicals, cosmetics and the like because water-soluble drugs can be encapsulated within the hollow globular lipid bilayer membrane of liposomes. In particular, application thereof to so-called drug delivery systems (DDS) has extensively been studied, wherein a drug, specifically an anticancer drug, is administered into living bodies followed by prolonged release of the drug encapsulated within liposomes, or wherein the surface of liposomes is modified to deliver drugs selectively to a target cancer site.

Various processes for the production of liposomes have been proposed. Examples thereof include reverse-phase evaporation method, sonication method, ethanol pour method, french press method, ether pour method, freeze thaw method and the like. In any of these processes, an aqueous solution of a drug is mixed with phospholipids, and a part of the aqueous drug solution is encapsulated within liposomes. Most of the aqueous drug solution is outside of the liposomes. Then, the un-encapsulated drug must be removed by dialysis or the like. Even large unilamellar vesicles (ca. 200nm) formed by reverse-phase evaporation method(reverse-phase evaporation vesicle, REV), whose encapsulating rate of the aqueous drug solution is considered to be the best, has an encapsulating rate of at most 30%. There is a limit to the amount of the drug to be utilized per a given amount of lipids. In particular, since many of anticancer drugs do not dissolve in solvents other than aqueous solvents and, in addition, the solubility in water is very low, the amount of the drug which can be encapsulated within liposomes is necessarily small.

In the case of cisplatin, for example, since it does not dissolve in solvents other than aqueous solvents and the saturated concentration in aqueous solvents is only about 2 mg/ml at ordinary temperature, the amount of cisplatin in liposomes obtained is at most 10 to 15 µg per 1 mg of lipids. Therefore, the dose of liposomes has to be increased in order to administer a certain amount of drug into living bodies. An increased amount of phospholipids constituting liposomes is required, and relatively excessive costs for the production are needed. Then, there are many unprofitable cases.

### OBJECTS OF THE INVENTION

The main object of the present invention is to provide a liposome composition in which a water-soluble drug is encapsulated in high concentration.

This object as well as other objects and advantages of the present invention will become apparent to those skilled in the art from the following description by reference to an accompanying drawing.

### BRIEF EXPLANATION OF THE DRAWING

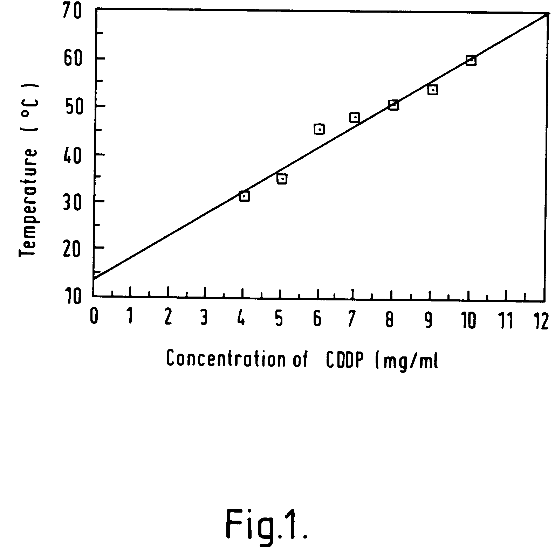

Fig. 1 shows a relationship between the temperature and the saturated solubility of cisplatin (CDDP) in physiological saline.

### SUMMARY OF THE INVENTION

The present inventors have intensively studied to solve the above problems in conventional liposomes. As a result, it has been found that, when a drug in liposomes is present in the supersaturated state or in the form of solids or crystals, the amount of the drug per unit lipid amount can be increased. Thus, the present invention has been completed.

That is, when the solubility of a drug in an aqueous solution increases with an increase of temperature and, if liposomes are prepared while maintaining a certain temperature or higher, the absolute quantity of the drug encapsulated within liposomes per lipid amount can necessarily be increased even though the encapsulating rate of the drug within liposomes is the same.

In this case, since the un-encapsulated drug within liposomes can be deposited by cooling the liposome solution, the drug can be readily recovered and re-used. Then, the loss of the drug in this case is the same as the amount of un-utilized drug at ordinary temperature in conventional methods.

Thus, according to the present invention, there is provided:
(1) A liposome composition comprising liposomes in which an aqueous solution containing a water-soluble drug in saturated concentration or more at ordinary temperature is encapsulated; and
(2) A process for producing a liposome composition which comprises forming a liposome membrane under warming by using as a solution to be encapsulated an aqueous solution containing a water-soluble drug in saturated concentration or more at ordinary temperature.

### DETAILED DESCRIPTION OF THE INVENTION

According to the present invention, liposomes containing a water-soluble drug in high concentration can be obtained by (i) preparing an aqueous solution of a water-soluble drug in an aqueous solvent [e.g., water, physiological saline, such solution (e.g., 5% glucose solution, etc.), sugar alcohol solution (e.g., 5% mannitol solution, etc.), a mixture thereof, etc.] at a given temperature of ordinary temperature or higher so that the aqueous solution contains the water-soluble drug in saturated concentration or higher at ordinary temperature, (ii) forming liposomes by conventional methods such as reverse-phase evaporation method, sonication method or the like while maintaining the solution at the given temperature or higher, and then (iii) removing the drug un-encapsulated within the liposomes for encapsulating the drug within the liposomes in high concentration. The ordinary temperature used herein is in the range of 15°C to 25°C as shown in the Pharmacopoeia of Japan.

Basically, any drugs which dissolve in water are applicable to the present invention without any limitation. Then, the water-solubility of the drug is not specifically limited. Preferably, the present invention is applicable to the uses of drugs whose solubility in water is generally 0.1 to 100 mg/ml, more preferably 0.5 to 50 mg/ml in the range of pH 3 to 9 at 25°C. The drugs used in the present invention are not specifically limited except those which become greatly unstable upon contact with phospholipids or dissolution at ordinary temperature or higher and those which form needles greatly in liposomes upon cooling to ordinary temperature. A drug having much lower solubility in an aqueous solvent at ordinary temperature produces more prominent effects. Examples of drugs which can be used in the present invention include anticancer drugs (e.g., cisplatin, carboplatin, etc.), antipyretics (e.g., aspirin, acetaminophen, indometacin, etc.), hormones (e.g., cortisone acetate, etc.) and the like. Among them, an anticancer drug such as cisplatin is preferably used in the present invention. However, the present invention is not limited to the drugs as described above.

The phospholipid to be used in the present invention may be any of (1) phospholipids whose diacyl group is saturated or unsaturated such as egg yolk lecithin, dimyristoylphosphatidyl choline, dipalmitoylphosphatidyl choline, distearoylphosphatidyl choline, dioleoylphosphatidyl choline and the like, (2) phospholipids whose acyl group is saturated or unsaturated and whose hydrophilic group is ethanolamine, serine, inositol or glycerol and (3) phospholipids whose acyl group is "lyso". These phospholipids can be used alone or in combination thereof.

The methods applicable to the production of the liposome composition of the present invention such as reverse-phase evaporation method or sonication method are not specifically limited, and per se known methods can be adopted. The present invention includes any methods in which liposomes can be prepared while maintaining ordinary temperature or higher. At this time, the temperature at which the drug dissolves is not specifically limited in so far as it is ordinary temperature or higher, preferably, 40°C or higher. The temperature can appropriately be selected depending upon the solubility of a particular drug, desired drug concentration, heat stability of the drug and the like. The maximum temperature is generally 100°C.

For example, the production by reverse-phase evaporation method is illustrated. In this method, a liposome starting solution can be obtained by (a) dissolving a drug in an aqueous solvent under warming with stirring, (b) maintaining a solution of a lipid dissolved in chloroform or the like at a given temperature, (c) mixing the drug solution and the lipid solution with a high-speed mixer while maintaining the mixture at a given temperature to obtain a W/O emulsion and (d) distilling off the solvent under reduced pressure with a rotary evaporator while warming the emulsion on a water bath and while maintaining the emulsion at a given temperature or higher. This starting solution is cooled to ordinary temperature, the deposited drug is removed by centrifugation or the like. Then, the drug un-encapsulated within the liposomes is removed by using a dialysis membrane to obtain a liposome solution containing the drug in high content.

In the present invention, the above (a) step is carried out by dissolving a water-soluble drug to be used in an aqueous solvent under warning to ordinary temperature or higher so that the drug is dissolved in an amount of more than the solubility of the drug in water at ordinary temperature. In the above (b), (c) and (d) steps, the temperature is also maintained at the warming temperature in the (a) step or higher to prepare liposomes.

In the present invention, the drug concentration in liposomes at the time of their formation is about the same as that in the external phase and the amount of the drug which can be encapsulated within liposomes formed by a certain amount of lipids can unequivocally be determined by the saturated solubility of the drug at the production temperature. Therefore, by taking this into consideration, if the amount of the drug initially dissolved is suitable selected according to a particular kind of drug and the purpose of use, the desired amount of the drug can be encapsulated within liposomes.

The concentration of the water-soluble drug is preferably 1.2 times or more the saturated concentration at ordinary temperature. For example, cisplatin is preferably used in a concentration of 1.2 to 12 times the saturated concentration at ordinary temperature.

In the production of liposomes, various additives (e.g., cholesterol, etc.) can be used according to per se known methods depending upon the purposes such as improvement of a liposome membrane.

Depending upon the particular drug encapsulated, the liposome composition of the present invention can be used according to the same manner as in conventional liposome compositions. However, since the concentration of the encapsulated drug is increased, the dose (the total amount of the pharmaceutical composition) can be reduced when it is administered.

According to the present invention, a liposome composition of a water-soluble drug having a high content of the drug per unit lipid amount can be obtained. Further, the desired effect can be obtained by a lower dose than in conventional methods. Furthermore, the costs for the production of the liposome composition can be reduced.

The following experiments and examples further illustrate the present invention in detail but they are not to be construed as limiting the scope of the present invention. In these experiments and examples, cisplatin (CDDP) is used as a drug.

However, the drug used in the present invention is not limited only to cisplatin and the present invention is applicable to any drug which is stable under warming. It is apparent that the present invention is applicable to any kinds of phospholipids constituting liposomes, any kinds of additives and the amount of the additive to be formulated.

### Experiment 1

Crystals of cisplatin (30 mg) and physiological saline (30 ml) were placed in a 100 ml glass beaker. The mixture was stirred on a water bath 60°C to dissolve the crystals. Then the solution was cooled to room temperature. Dipalmitoylphosphatidyl choline (600 mg) and chloroform (100 ml) were placed in a 300 ml glass eggplant type flask. After dissolution of the dipalmitoylphosphatidyl choline, isopropyl ether (100 ml) was added. The above cisplatin solution was added to the resulting mixture. A W/O type emulsion was obtained by using a high-speed emulsifier. The emulsion was placed in a 5 liter eggplant type flask. The organic solvent was distilled off under reduced pressure with a rotary evaporator under warming on a water bath at 60°C to obtain a liposome solution (20 ml). The amount of encapsulated cisplatin per unit phospholipid was 10 µg/mg.

This method is called reverse-phase evaporation method, by which large unilamellar vesicles (LUV) (ca. 200nm) having a single membrane can be produced efficiently and it is considered that liposomes including the highest amount of the drug can be obtained.

The liposome solution (20 ml) was placed in a dialysis bag [molecular weight cut off: 8000, SPECTORA POR (trade mark)] and dialyzed in physiological saline overnight to remove cisplatin un-encapsulated within the liposomes. The cisplatin content in the liposome solution thus obtained was 300 µg/ml, and the content of cisplatin un-encapsulated within the liposomes was 2 µg/ml.

### Example 1

Crystals of cisplatin (240 mg) were dissolved in physiological saline (30 ml) at 60°C with stirring. Dipalmitoylphosphatidyl choline (600 mg) was dissolved in chloroform (100 ml), and isopropyl ether (100 ml) was added and the mixture was warmed to 60°C. To this mixture was added the above cisplatin solution. A W/O emulsion was obtained on a water bath at 60°C by using a high speed emulsifier. This emulsion was placed in a 5 liter eggplant type flask. The organic solvent was distilled off under reduced pressure with a rotary evaporator under warning on a water bath at 60°C to obtain a liposome solution (20 ml). The liposome solution (20 ml) was cooled to room temperature. The crystallized cisplatin crystals were separated and removed with a centrifugal separator. The solution was placed in a dialysis bag (molecular weight cut off: 8000, SPECTORA POR) and dialyzed in physiological saline overnight to remove cisplatin un-encapsulated within the liposomes. The cisplatin crystals removed by centrifugation were re-used. The cisplatin content in the liposome solution thus obtained was 2.6 mg/ml, and the content of cisplatin un-encapsulated within the liposomes was 3 µg/ml. The amount of encapsulated cisplatin per unit phospholipid was 87 µg/mg.

### Experiment 2

According to the same manner as that described in Experiment 1, LUV was prepared by REV method. A liposome solution (ca. 100 nm) was obtained by using a probe type sonicator, and then the solution was cooled to room temperature. The solution was placed in a dialysis bag (molecular weight cut off: 8000, SPECTORA POR) and dialyzed in physiological saline overnight to remove cisplatin un-encapsulated within the liposomes. In 20 ml of the liposome solution thus obtained, the cisplatin content was 17 µg/ml, and the content of cisplatin un-encapsulated within the liposomes was 1 µg/ml. The amount of encapsulated cisplatin per unit phospholipid was 0.6 µg/mg.

### Example 2

According to the same manner as that described in Example 1, LUV liposomes were obtained by REV method. From the LUV liposomes, liposomes of about 100 nm were prepared with a probe type sonicator while maintaining them at 60°C. The liposomes were cooled to room temperature. After removal of the deposited crystals of cisplatin by centrifugation, the crystals were placed in a dialysis bag (molecular weight cut off: 8000, SPECTORA POR) and dialyzed in physiological saline overnight to remove cisplatin un-encapsulated within the liposomes. In 20 ml of the liposome solution thus obtained, the cisplatin content was 190 µg/ml, and the content of cisplatin un-encapsulated within the liposomes was 3 µg/ml. The cisplatin crystals removed by centrifugation were re-used. The amount of encapsulated cisplatin per unit phospholipid was 6.3 µg/mg.

### Experiment 3

Cisplatin (10 mg) was dissolved in physiological saline (10 ml) at ordinary temperature. Dipalmitoylphosphatidyl choline (200 mg) and cholesterol (15 mg) were dispersed with a sonicator until the solution became transparent. The solution was cooled and dialyzed under the same conditions as those of Example 1. The diameter of the liposomes thus obtained was about 100 nm. The content of cisplatin encapsulated within the liposomes was 42 µg/ml, and the content of un-encapsulated cisplatin was 2 µg/ml. The amount of encapsulated cisplatin per unit phospholipid was 2.1 µg/mg.

### Example 3

Cisplatin (100 mg), dipalmitoylphosphatidyl choline (200 mg), cholesterol (15 mg) and physiological saline (10 ml) were placed in a 30 ml glass beaker. The mixture was warmed to 80°C with stirring with a magnetic stirrer to dissolve the cisplatin. The lipid was swelled and dispersed. The solution was dispersed with a sonicator under warming until the solution became transparent, followed by removal of cisplatin deposited by cooling and centrifugation. The liposome solution was dialyzed under the same conditions as those of Example 1 to remove cisplatin un-encapsulated within the liposomes. The diameter of the liposomes thus obtained was about 100 nm. The content of cisplatin encapsulated within the liposomes was 178 µg/ml, and the content of un-encapsulated cisplatin was 9 µg/ml. The amount of encapsulated cisplatin per unit phospholipid was 8.9 µg/mg.

## Claims

1. A liposome composition comprising liposomes in which an aqueous solution containing a water-soluble drug in saturated concentration or more at ordinary temperature is encapsulated.

2. A liposome composition according to claim 1 wherein the water-soluble drug has a solubility in water of 0.1 to 100 mg/ml at pH 3 to 9 at 25°C.

3. A liposome composition according to claim 1, wherein the water-soluble drug is selected from the group consisting of anticancer drugs, antipyretics and hormones.

4. A liposome composition according to claim 3, wherein the water-soluble drug is an anticancer drug.

5. A liposome composition according to claim 4, wherein the water-soluble drug is cisplatin.

6. A liposome composition according to claim 1, wherein the aqueous solution is physiological saline.

7. A process for producing a liposome composition which comprises forming a liposome membrane under warming by using as a solution to be encapsulated an aqueous solution containing a water-soluble drug in saturated concentration or more at ordinary temperature.

8. A process according to claim 7, wherein the liposome membrane is formed under warming to 40°C or higher.

9. A process according to claim 7, wherein the water-soluble drug has a solubility in water of 0.1 to 100 mg/ml at pH 3 to 9 at 25°C.

10. A process according to claim 7, wherein the water-soluble drug is selected from the group consisting of anticancer drugs, antipyretics and hormones.

11. A process according to claim 10, wherein the water-soluble drug is an anticancer drug.

12. A process according to claim 11, wherein the water-soluble drug is cisplatin.

13. A process according to claim 7, wherein the aqueous solution is physiological saline.

14. A process according to claim 7, wherein the liposomes are prepared by reverse-phase evaporation method.
